(19) Europäisches Patentamt
European Patent Office
Office européen des brevets

(11) EP 3 307 848 B1

(12) **EUROPEAN PATENT SPECIFICATION**

(45) Date of publication and mention of the grant of the patent:
**06.03.2019 Bulletin 2019/10**

(21) Application number: **16723021.8**

(22) Date of filing: **17.05.2016**

(51) Int Cl.:
***C07C 69/734*** (2006.01) ***C07C 69/76*** (2006.01)
***C09K 19/58*** (2006.01) ***C09K 19/32*** (2006.01)
***C09K 19/30*** (2006.01) ***C09K 19/20*** (2006.01)
***C09K 19/12*** (2006.01)

(86) International application number:
**PCT/EP2016/000811**

(87) International publication number:
**WO 2016/198143 (15.12.2016 Gazette 2016/50)**

(54) **CHIRAL DOPANTS HAVING A NORTRICYCLAN UNIT**

CHIRALE DOTIERSTOFFE MIT NORTRICYCLAN-EINHEIT

DOPANTS CHIRAUX COMPORTANT UNE UNITÉ NORTRICYCLANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.06.2015 EP 15001716**

(43) Date of publication of application:
**18.04.2018 Bulletin 2018/16**

(73) Proprietor: **Merck Patent GmbH**
**64293 Darmstadt (DE)**

(72) Inventors:
- **KIRSCH, Peer**
  **64342 Seeheim-Jugenheim (DE)**
- **HAUFE, Guenter**
  **48163 Muenster (DE)**
- **KOZEL, Volodymyr**
  **Kiev 02095 (UA)**
- **GRAJEWSKI, Jakub**
  **Poznan 60-688 (PL)**

(56) References cited:
**EP-A1- 2 309 319**
**WO-A1-03/002513**
**WO-A1-03/002515**
**WO-A1-03/002516**
**US-A1- 2012 105 793**

- **HOURS A E ET AL: "PtCl2-Promoted cyclopropane opening in [4+2+2] homo Diels-Alder cycloadducts", TETRAHEDRON LETTERS, PERGAMON, GB, vol. 47, no. 5, 30 January 2006 (2006-01-30), pages 675-678, XP025003217, ISSN: 0040-4039, DOI: 10.1016/J.TETLET.2005.11.109 [retrieved on 2006-01-30]**
- **PANKAJ P. DARAMWAR ET AL: "Biocatalyst mediated regio- and stereo-selective hydroxylation and epoxidation of (Z)-[alpha]-santalol", ORGANIC & BIOMOLECULAR CHEMISTRY, vol. 12, no. 7, 1 January 2014 (2014-01-01), page 1048, XP055289427, GB ISSN: 1477-0520, DOI: 10.1039/c3ob42174k**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 307 848 B1

**Description**

The present invention relates to chiral dopants having a nortricyclan unit and to liquid-crystalline media which comprise these compounds. The present invention furthermore relates to liquid-crystal displays which contain the liquid-crystal mixtures according to the invention.

In the known liquid-crystal displays, the liquid crystals, generally liquid-crystalline mixtures, are used as dielectrics whose optical properties change reversibly on application of an electric voltage. These liquid-crystal displays use various electro-optical effects. The commonest of these are the TN (twisted nematic) effect, with a homogeneous, virtually planar initial alignment of the liquid crystals and a nematic structure twisted by about 90°, the STN (supertwisted nematic) effect and the SBE (supertwisted birefringence effect), both of which, like the TN effect, use a twisted, homogeneous initial alignment of the liquid crystals, but here the molecules have a significant surface tilt angle ("tilt angle" for short) at the surface of the substrates, and the twist between the substrates is significantly greater than 90°. In this application, unless explicitly stated otherwise, the STN effect and the SBE below are both jointly referred to as the STN effect. The tilt angle at the surface in STN displays is typically between 2° and 10°. It is greater the greater the twist angle. The twist angles are generally from about 180° to 240°, sometimes also up to 260° or 270° and in some cases even greater.

The twist of the liquid-crystal medium by greater than 90° is achieved through the use of chiral liquid-crystal mixtures whose natural twist is selected in accordance with the layer thickness of the liquid-crystal layer. To this end, two possibilities are available to the person skilled in the art. The first consists in the use of liquid crystals which are themselves chiral, i.e. cholesteric liquid crystals. Such liquid crystals themselves have a twisted structure. In a homogeneously oriented arrangement between two substrates, which is known as the Grandjean texture, the director of the molecules is helically twisted in the vertical direction, i.e. over the thickness of the layer.

The characteristic length for a complete rotation through 360° is known as the cholesteric pitch (P). However, the use of cholesteric liquid crystals is often not particularly advantageous since the cholesteric pitch of cholesteric liquid crystals cannot be matched easily to the layer thicknesses of the display cells usually used. In addition, the cholesteric pitch of these liquid crystals is often disadvantageously and in many cases strongly dependent on the temperature. A change in the composition of the mixtures also usually results in considerable changes in the cholesteric pitch.

For this reason, in most practical cases a chiral substance which induces the desired twist is added to a nematic liquid-crystal mixture. It is not particularly important here whether this compound itself has a mesophase. Rather, it is more important that it has a high twisting power for the nematic base mixture (also known as host mixture) and that it does not significantly deteriorate the advantageous properties of the base mixture, in particular its clearing point in the concentrations usually employed. Preference is thus generally given to compounds which themselves have a mesogenic structure or are even cholesteric.

The cholesteric phases which are induced by addition of chiral substances to nematic liquid crystals are often known as chiral nematic phases. In the present application, however, these are also referred to as cholesteric phases unless explicitly stated otherwise.

The cholesteric pitch induced by addition of chiral substances (dopants) to nematic liquid crystals is dependent at a given temperature, besides on the enantiomeric purity of the chiral dopant, in particular on the dopant concentration (c) employed and on the twisting power of the dopant. This twisting power is known as the HTP (helical twisting power). To a first approximation, the induced cholesteric pitch (P) is inversely proportional to the product of HTP and dopant concentration employed, as shown in equation (1).

$$P = (HTP \cdot c)^{-1} \qquad (1)$$

In STN displays, use is typically made of liquid-crystal mixtures having a cholesteric pitch to layer thickness ratio (d/P) in the range from 0.4 to 0.8, frequently of about 0.5.

However, chiral liquid-crystal mixtures are also used in TN displays, here in order to avoid twist in the reverse direction (reverse twist). Occurrence of this would result in the formation of domains and thus in a reduction in contrast. In TN displays, use is generally made of cholesteric liquid-crystal mixtures having a significantly smaller d/P ratio than in STN displays since larger d/P values in most cases result in an increase in the threshold voltage. The values here are typically from about 0.01 to 0.3, frequently about 0.1.

Besides these display types, there are further liquid-crystal displays which use liquid-crystal mixtures doped with chiral compounds, like for example LCDs utilising the electro-optical blue phase as a switching medium, or surface-stabilised cholesteric texture (SSCT) displays.

A cheap, stable, chiral polymerisable dopant is an essential component for the mass production of cholesteric and other chiral optical polymer films made from reactive mesogens for display applications. A chiral dopant is also

required for example in the mass production of security films made from reactive mesogens.

Known chiral dopants are, for example, the compounds C15, CB15, R-811 and S-811, R-1011 and S-1011, R-5011 and S-5011, and R-2011 and S-2011, all Merck KGaA.

Chiral compounds having a nortricyclan unit are also known in the art, see A. E. Hours, J. K. Snyder, "PtCl2-Promoted cyclopropane opening in [4+2+2] homo Diels-Alder cycloadducts", TETRAHEDRON LETTERS, 47 (2006), 675-678, for instance.

In STN and TN displays, use is regularly made of liquid-crystalline media of positive dielectric anisotropy ($\Delta\varepsilon$). Besides the electro-optical effects mentioned above, which require liquid-crystal media of positive dielectric anisotropy, there are other electro-optical effects which use liquid-crystal media of negative dielectric anisotropy, such as, for example, the ECB (electrically controlled birefringence) effect and its sub-forms DAP (deformation of aligned phases), VAN (vertically aligned nematics) and CSH (colour super homeotropics). In these and similar electro-optical effects, use is made of liquid-crystalline media of negative dielectric anisotropy ($\Delta\varepsilon$).

An electro-optical effect having excellent, low viewing-angle dependence of the contrast uses axially symmetrical micropixels (ASMs). In this effect, the liquid crystal of each pixel is surrounded cylindrically by a polymer material. This mode is particularly suitable for combination with addressing through plasma channels. In particular, large-area PA LCDs having good viewing-angle dependence of the contrast can be achieved in this way.

The IPS (in plane switching) effect employed to an increased extent recently can use both dielectrically positive and dielectrically negative liquid-crystal media, similar to guest-host displays, which can employ dyes either in dielectrically positive or in dielectrically negative media, depending on the display mode used.

The pixels of the liquid-crystal displays can be addressed directly, time-sequentially, i.e. in time-multiplex mode, or by means of a matrix of active, electrically non-linear elements.

The most common AMDs (active matrix displays) hitherto use discrete active electronic switching elements, such as, for example, three-pole switching elements, such as MOS (metal oxide silicon) transistors or thin-film transistors (TFTs) or varistors or 2-pole switching elements, such as, for example, MIM (metal insulator metal) diodes, ring diodes or back-to-back diodes. In the TFTs, various semiconductor materials, predominantly silicon or alternatively cadmium selenide, are used. In particular, amorphous silicon or polycrystalline silicon is used.

In prior art DE 43 22 905 A1 discloses chiral 2,6-difluorobenzene derivatives of the formula:

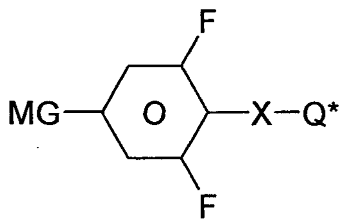

in which

MG is a mesogenic group which is free from ester groups,
X is O or $CH_2$, and
Q* is a chiral radical having at least one chiral carbon atom.

The use of these derivatives as dopant in tilted smectic liquid-crystal phases results, even when added in small amounts, in strong twisting in the cholesteric phase. Electro-optical displays containing these liquid-crystalline media are also disclosed.

EP 0 609 566 A1 likewise discloses liquid-crystalline compositions which comprise chiral compounds of the formula

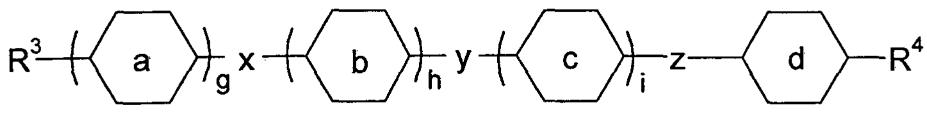

in which the rings

a, b, c and d, independently of one another, are trans-1,4-cyclohexylene, 1-cyclohexene-1,4-diyl or 1,4-phenylene,

g, h and i are each 0 or 1, where $(g + h + i) \geq 1$,

x, y and z, independently of one another, are a single bond, $-CH_2CH_2-$, -CH=CH-, -C≡C-, $-OCH_2-$ or $-CH_2O-$,

$R^3$ and $R^4$, independently of one another, are H, $C_nH_{2n+1}-$, $C_nH_{2n+1}-O-$, $C_nH_{2n+1}-O-C_kH_{2k}-$, $C_nH_{2n-1}-$, $C_nH_{2n-1}-O-$, $C_nH_{2n-1}-O-C_kH_{2k}-$, $C_nH_{2n-3}-$, $C_nH_{2n-3}-O-$ or $C_nH_{2n-3}-O-C_kH_{2k}-$, and

n and k, independently of one another, are an integer from 1 to 18, where $(n + k) \leq 18$,

where at least one H atom in the formula may be replaced by an F atom, and at least one of the radicals $R^3$, $R^4$, x, y and z has an asymmetric carbon atom.

The chiral dopants known from the prior art have the disadvantage that they only result in inadequate twisting in LC mixtures and in addition may adversely affect the properties of the LC mixtures.

Based on the cited prior art, it can be regarded as the object of the present invention to provide novel chiral dopants, in particular for TFT-LC mixtures, which are superior to the prior art and which preferably do not adversely affect the properties of the LC material.

Surprisingly, it has been found that the dopants according to the invention have an advantageous helical twisting power (HTP), high solubility in conventional liquid crystalline mixtures and high stability against light The present invention thus relates to chiral dopants having a nortricyclan unit of the general formula I:

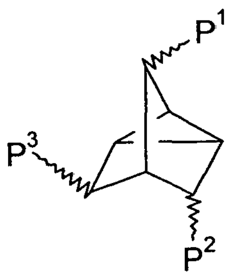

(I)

in which:

$P^1$, $P^2$ and $P^3$ independently, are a group $R-(A^2-Z^2)_n-A^1-Z^1-$,

R is, independently, -H, an alkyl or alkenyl radical having from 1 to 12 carbon atoms which is unsubstituted or at least monosubstituted by halogen, and in which one or more non-adjacent $-CH_2-$ groups may be replaced by -O- or -S- and/or -C≡C-, as well as F or Cl,

$A^1$, independently, is a 1,4-phenylene, in which one, two or three CH groups may be substituted by N and in which, in addition, one or more H atoms may be replaced by F, 1,4-cyclohexylene, in which, in addition, one or two $CH_2$ groups may be replaced by -O-, or 1,4-bicyclo[2.2.2]octanyl,

$A^2$, independently, is 1,4-phenylene, in which one, two or three CH groups may be substituted by N and in which, in addition, one or more H atoms may be replaced by F, 1,4-cyclohexylene, in which, in addition, one or two $CH_2$ groups may be replaced by -O-, or 1,4-bicyclo[2.2.2]octanyl,

$Z^1$, independentlyis a single bond, -O-, -S-, -CO-, -COO-, -OCO-, -S-CO-, -CO-S-, -O-COO-, $-CH_2-CH_2-$, -CH=CH-, $-CH=CR^0-$, $-CR^0=CR^{00}-$, $-O-CH_2-$, $-CH_2-O-$, $-CF_2-O-$, $-O-CF_2-$, $-CF_2-CF_2-$ or -C≡C-, -CH=CH-COO- or -OCO-CH=CH-, preferably -O-, $-CF_2-O-$or -COO-, more preferably -O-,

$Z^2$, independently is a single bond, -O-, -S-, -CO-, -COO-, -OCO-, -S-CO-, -CO-S-, -O-COO-, $-CH_2-CH_2-$, -CH=CH-, $-CH=CR^0-$, $-CR^0=CR^{00}-$, $-O-CH_2-$, $-CH_2-O-$, $-CF_2-O-$, $-O-CF_2-$, $-CF_2-CF_2-$ or -C≡C-, -CH=CH-COO- or -OCO-CH=CH-, preferably a single bond,

$R^0$ and $R^{00}$ are independently of each other H or alkyl with 1 to 12 C-atoms, F, Cl or CN, and

n is 0, 1, 2 or 3, preferably 0 or 1.

The oscillating bonds of the groups $P^1$, $P^2$ and $P^3$ shall indicate any kind of stereochemistry (R or S) at the involved carbon atom.

Preference is given to chiral dopants having the following basic structures (Ia) and (Ib):

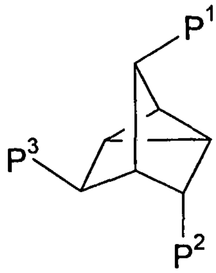

(Ia)

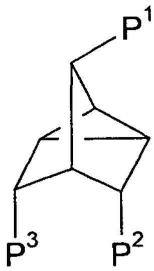

(Ib)

where $P^1$ to $P^3$ are defined as in for (I), and the bonds indicate a specific stereostructure. The structure (Ia) has a $C_3$ symmetry, not taking into account any differences in P substitutents, and structure (Ib) has a $C_1$ symmetry.

Where stereopecific structures comprise enantiomers (chiral compounds) the structures generally include both enantiomers, unless a specific enantiomer is specified in the context of the drawing. For example the structure (Ia) can comprise the structures

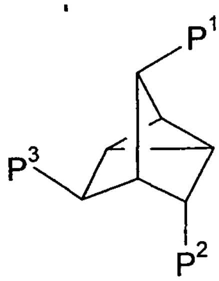

and

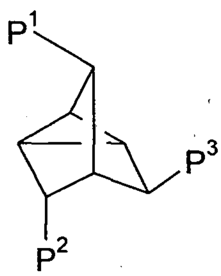

.

For the application of the compounds, e.g. as chiral dopants, the compounds are preferably used in enantiomerically pure, almost pure or at least enriched form.

In the general formulae above and below the groups $P^1$, $P^2$ and $P^3$, independently, preferably denote a group selected from the following:

$R\text{-}A^2\text{-}A^1\text{-}Z^1\text{-}$,

$R\text{-}A^2\text{-}A^2\text{-}A^1\text{-}Z^1\text{-}$,

or

$R\text{-}A^2\text{-}Z^2\text{-}A^1\text{-}Z^1\text{-}$.

In the structures and substructures of formula (I) above and below preferably the groups $A^1$ and $A^2$ are, independently, selected from the following structures:

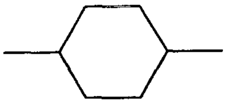 , 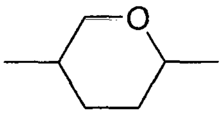 , 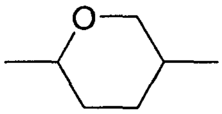 , 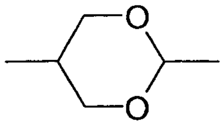 ,

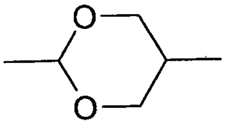 , 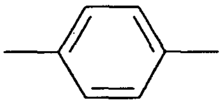 , 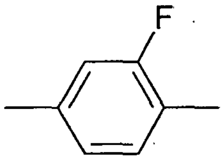 , 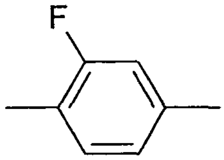 ,

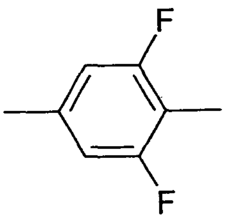 , 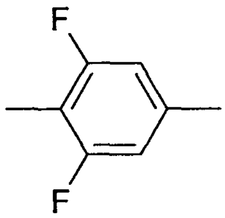

or

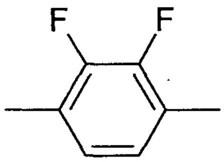 ,

more preferably

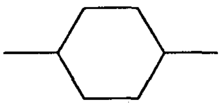 , 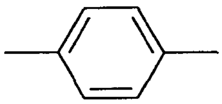 , 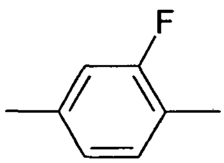 ,

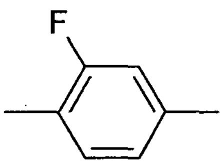

or

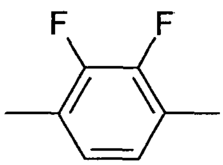 .

In the structures and substructures of formula (I) above and below preferably the group $Z^1$ is preferably a group

with a partial function -O-, which is directly connected to the chiral nortricyclan. $Z^1$ is more preferably -O-, -COO-, or $-CF_2O-$, more preferably -O- or -COO-.

In the structures and substructures of formula (I) above and below the group $Z^2$ is preferably a single bond, $-OCF_2-$ or $-CF_2O-$, and more preferably a single bond.

Among the general formulae (I) or (Ia) and (Ib) the following structures are particularly preferred:

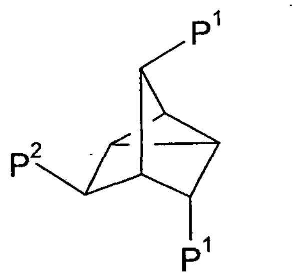

(Iaa)

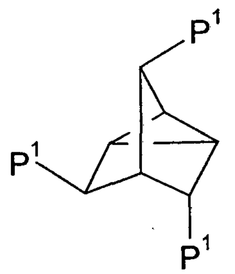

(Iab)

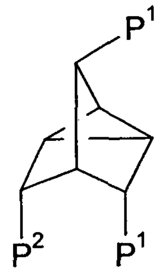

(Iba)

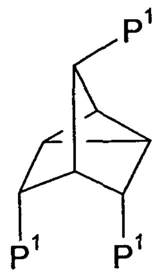

(Ibb)

wherein the groups $P^1$ are defined as for structure (I) and indicate the same group at each occurrence, and $P^2$ are defined as for structure (I) and indicate a group which is the same or different from $P^1$. Particular preference is given to structures (Iab) and (Ibb) depicted above, which have three identical groups $P^1$.

The partial structure $R-(A^2-Z^2)_n-$ in any of $P^1$ to $P^3$ is preferably not a single H atom. Each $P^1$ to $P^3$ is defined independently, but most preferable all $P^1$ to $P^3$ are identical. Further, $R-(A^2-Z^2)_n-$ is preferably selected from the following partial structures:

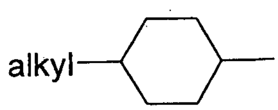

(1)

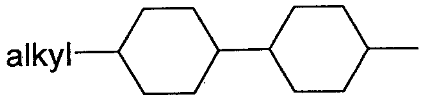

(2)

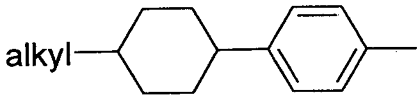

(3)

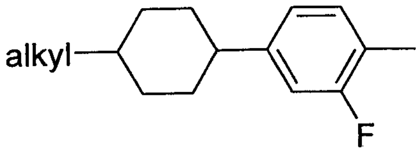

(4)

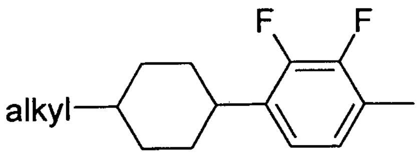

(5)

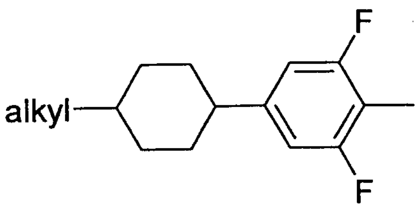

(6)

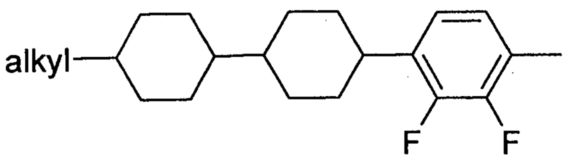

(7)

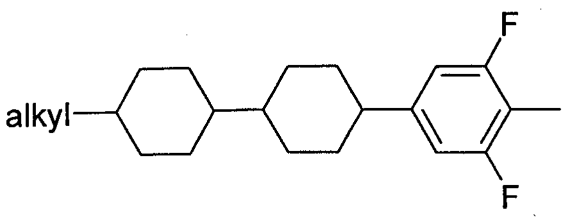

(8)

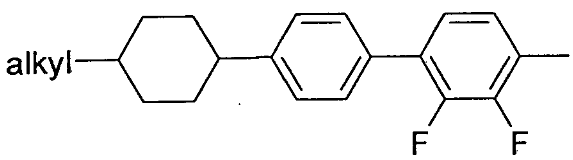

(9)

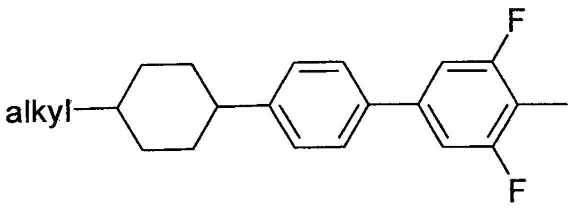

(10)

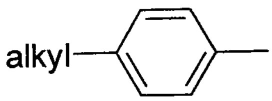

(11)

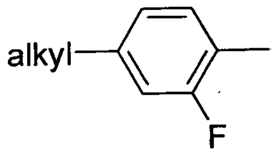

(12)

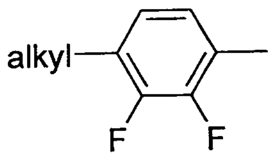

(13)

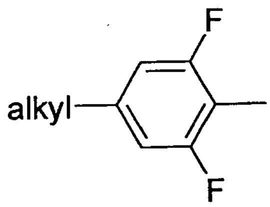

(14)

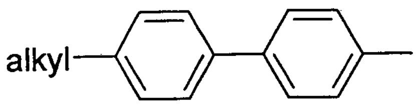

(15)

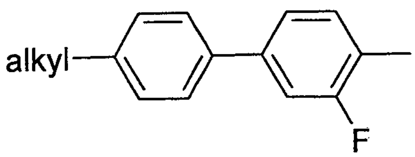

(16)

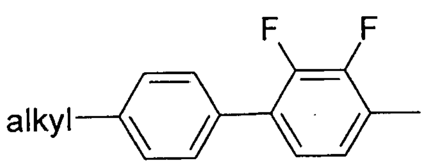

(17)

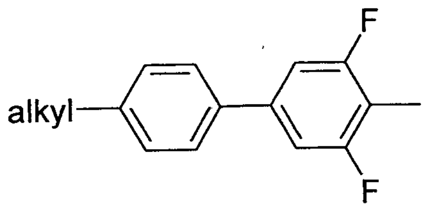

(18)

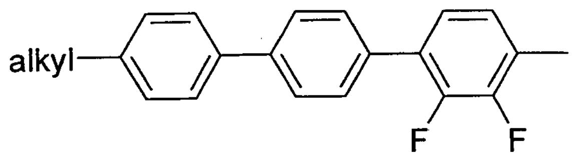

(19)

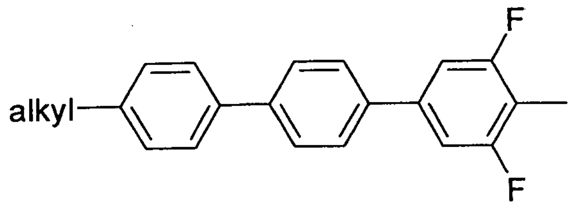

(20)

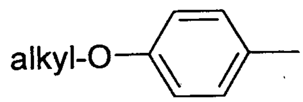

(21)

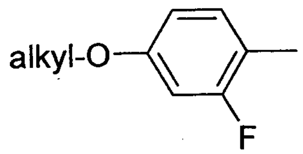

(22)

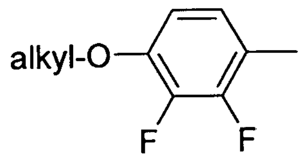

(23)

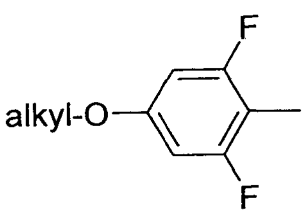

(24)

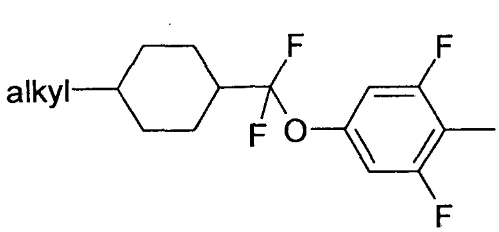

(25)

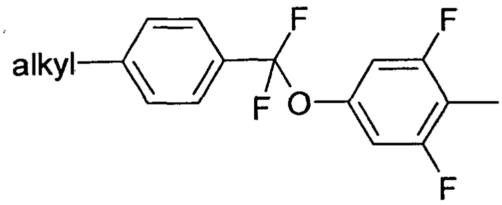

(26)

where alkyl is an alkyl radical having from 1 to 12 carbon atoms, which may be straight-chain or branched. It is preferably straight-chain, has 1, 2, 3, 4, 5, 6 or 7 carbon atoms and accordingly is preferably methyl, ethyl, propyl, butyl, pentyl, hexyl or heptyl;

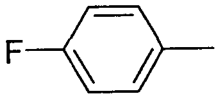

(27)

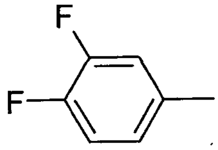

(28)

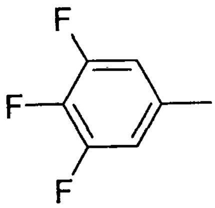

(29)

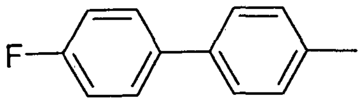

(30)

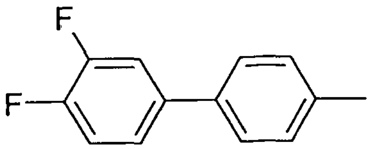

(31)

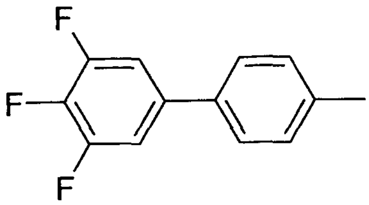

(32)

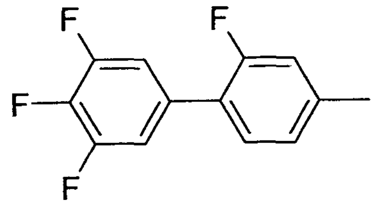

(33)

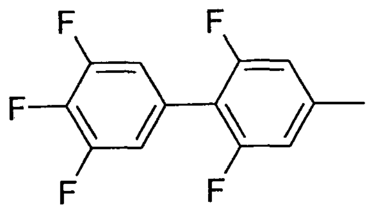

(34)

Unit $R-(A^2-Z^2)_n$ is particularly preferably selected from groups (2) to (10), (15) to (20) and (28) to (32) mentioned and in particular groups (3), (5), (7), (9), (11), (15), (17), (19), (27) and (30).

Compounds that are more specific are derivable from the exemplified compounds.

The compounds of the formula I are prepared by methods known per se, as described in the literature (for example in the standard works, such as Houben-Weyl, Methoden der Organischen Chemie [Methods of Organic Chemistry], Georg-Thieme-Verlag, Stuttgart), to be precise under reaction conditions which are known and suitable for the said reactions. Use can also be made here of variants which are known per se, but are not mentioned here in detail.

The particularly preferred compounds of the formula (I) in which $Z^1$ is -COO-, the ethereal -O- connected to the nortricyclan structure, are prepared as shown in scheme 1:

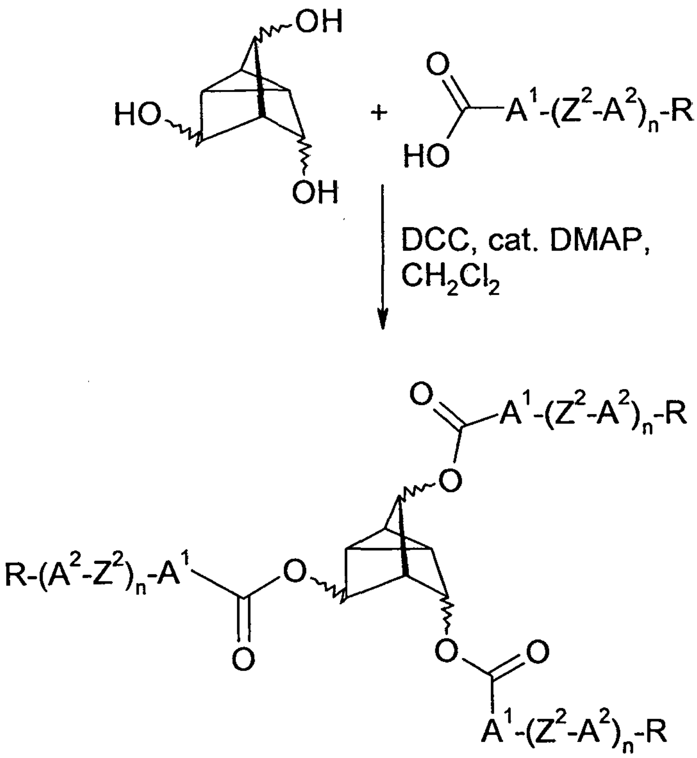


Scheme 1. Exemplary preparation of typical compounds according to the invention.

The tricyclo[2.2.1.$0^{2,6}$]heptan-3,5,7-triols (nortricycloheptanes) used as a starting material are prepared in a racemic manner and separated into stereoisomers by common resolution techniques, like e.g. derivatization with chiral acids and separation of diastereomers, or by chiral chromatography. A synthetic procedure to obtain the racemate and

single enantiomers of structures (Ia) and (Ib) is provided in the experimental part.

Alternatively, the racemic nortricycloheptanes are used for the synthesis of desired compounds, followed by enantiomeric resolution. In this case resolution of enantiomers can be effected by chromatography on a chiral stationary phase e.g. by chiral HPLC.

The compounds of the formula I are used as components of liquid-crystalline phases, preferably nematic, liquid-crystalline phases, for example for preventing reverse twist in TN displays.

The compounds of the formula I are particularly preferably employed as dopants for nematic, liquid-crystalline phases for STN and active-matrix displays. They are particularly distinguished here by a high helical twisting power (HTP) and by high voltage holding ratios. In particular, doped nematic mixtures of this type can easily be purified by treatment with aluminium oxide, with no or virtually no loss of chiral dopant occurring.

Furthermore, the chiral derivatives according to the invention can be used to prepare liquid-crystalline media for so-called "phase change" displays (for example Y. Yabe et al., SID 1991 Digest 261-264), for displays working in the so-called blue mode or for the preparation of polymerized cholesteric liquid crystal films or layers, i.e. with a helically twisted structure.

The present invention thus furthermore also relates to the use of the chiral dopants according to the invention in liquid-crystalline mixtures, preferably for producing twist.

The present invention thus likewise relates to liquid-crystalline mixtures comprising at least one chiral dopant of the formula I according to the invention.

The liquid-crystalline mixtures according to the invention comprise from about 0.01 to 30% by weight, preferably from 0.05 to 10% by weight, particularly preferably from 0.1 to 10% by weight and in particular from 0.1 to 5% by weight, of one or more compounds of the formula I.

The most important compounds suitable as further constituents of media according to the invention can be characterised by the formulae 1, 2, 3, 4 and 5:

R'-L-E-R" 1

R'-L-COO-E-R" 2

R'-L-OOC-E-R" 3

R'-L-$CH_2CH_2$-E-R" 4

R'-L-C≡C-E-R" 5

In the formulae 1, 2, 3, 4 and 5, L and E, which may be identical or different, are each, independently of one another, a divalent radical from the group formed by -Phe-, -Cyc-, -Phe-Phe-, -Phe-Cyc-, -Cyc-Cyc-, -Pyr-, -Dio-, -G-Phe- and -G-Cyc- and their mirror images, where Phe is unsubstituted or fluorine-substituted 1,4-phenylene, Cyc is trans-1,4-cyclohexylene or 1,4-cyclohexylene, Pyr is pyrimidine-2,5-diyl or pyridine-2,5-diyl, Dio is 1,3-dioxane-2,5-diyl, and G is 2-(trans-1,4-cyclohexyl)ethyl, pyrimidine-2,5-diyl, pyridine-2,5-diyl or 1,3-dioxane-2,5-diyl.

One of the radicals L and E is preferably Cyc, Phe or Pyr. E is preferably Cyc, Phe or Phe-Cyc. The media according to the invention preferably comprise one or more components selected from the compounds of the formulae 1, 2, 3, 4 and 5 in which L and E are selected from the group consisting of Cyc, Phe and Pyr and simultaneously one or more components selected from the compounds of the formulae 1, 2, 3, 4 and 5 in which one of the radicals L and E is selected from the group consisting of Cyc, Phe and Pyr and the other radical is selected from the group consisting of -Phe-Phe-, -Phe-Cyc-, -Cyc-Cyc-, -G-Phe- and -G-Cyc-, and optionally one or more components selected from the compounds of the formulae 1, 2, 3, 4 and 5 in which the radicals L and E are selected from the group consisting of -Phe-Cyc-, -Cyc-Cyc-, -G-Phe- and -G-Cyc-.

R' and/or R" are each, independently of one another, alkyl, alkenyl, alkoxy, alkoxyalkyl, alkenyloxy or alkanoyloxy having up to 8 carbon atoms, -F, -Cl, -CN, -NCS or $-(O)_iCH_{3-(k+l)}F_kCl_l$, where i is 0 or 1 and k and l are 1, 2 or 3.

In a smaller sub-group of the compounds of the formulae 1, 2, 3, 4 and 5, R' and R" are each, independently of one another, alkyl, alkenyl, alkoxy, alkoxyalkyl, alkenyloxy or alkanoyloxy having up to 8 carbon atoms. This smaller sub-group is called group A below, and the compounds are referred to by the sub-formulae 1a, 2a, 3a, 4a and 5a. In most of these compounds, R' and R" are different from one another, one of these radicals usually being alkyl, alkenyl, alkoxy or alkoxyalkyl.

In another smaller sub-group of the compounds of the formulae 1, 2, 3, 4 and 5, which is known as group B, R" is -F, -Cl, -NCS or $-(O)_iCH_{3-(k+l)}F_kCl_l$, where i is 0 or 1, and k and l are 1, 2 or 3; the compounds in which R" has this meaning are referred to by the sub-formulae 1b, 2b, 3b, 4b and 5b. Particular preference is given to those compounds of the sub-formulae 1b, 2b, 3b, 4b and 5b in which R" is -F, -Cl, -NCS, $-CF_3$, $-OCHF_2$ or $-OCF_3$.

In the compounds of the sub-formulae 1b, 2b, 3b, 4b and 5b, R' is as defined for the compounds of the sub-formulae 1a to 5a and is preferably alkyl, alkenyl, alkoxy or alkoxyalkyl.

In a further smaller sub-group of the compounds of the formulae 1, 2, 3, 4 and 5, R" is -CN; this sub-group is referred to below as group C, and the compounds of this sub-group are correspondingly described by sub-formulae 1c, 2c, 3c, 4c and 5c. In the compounds of the sub-formulae 1c, 2c, 3c, 4c and 5c, R' is as defined for the compounds of the sub-formulae 1a to 5a and is preferably alkyl, alkoxy or alkenyl.

Besides the preferred compounds of groups A, B and C, other compounds of the formulae 1, 2, 3, 4 and 5 having other variants of the proposed substituents are also customary. All these substances are obtainable by methods which are known from the literature or analogously thereto.

Preference is given to liquid-crystalline mixtures comprising at least one chiral dopant of the formula I according to the invention and at least one liquid-crystalline compound of the formulae 1, 2, 3, 4 and/or 5.

Besides compounds of the formula I according to the invention, the media according to the invention particularly preferably comprise one or more compounds selected from groups A, B and/or C.

The proportions by weight of the compounds from these groups in the media according to the invention are preferably

| | |
|---|---|
| group A: | from 0 to 90%, preferably from 20 to 90%, in particular from 30 to 90%, |
| group B: | from 0 to 80%, preferably from 10 to 80%, in particular from 10 to 65%, |
| group C: | from 0 to 80%, preferably from 5 to 80%, in particular from 5 to 50%, |

where the sum of the proportions by weight of the group A and/or B and/or C compounds present in the respective media according to the invention are preferably from 5% to 90% and particularly preferably from 10% to 90%.

The liquid-crystal mixtures which can be used in accordance with the invention are prepared in a manner which is conventional per se. In general, the desired amount of the components used in lesser amount is dissolved in the components making up the principal constituent, advantageously at elevated temperature. It is also possible to mix solutions of the components in an organic solvent, for example in acetone, chloroform or methanol, and to remove the solvent again, for example by distillation, after thorough mixing. It is furthermore possible to prepare the mixtures in other conventional manners, for example by using premixes, for example homologue mixtures, or using so-called "multi-bottle" systems.

The dielectrics may also comprise further additives known to the person skilled in the art and described in the literature. For example, 0-15%, preferably 0-10%, of pleochroic dyes and/or additional chiral dopants can be added. The individual compounds added are employed in concentrations of from 0.01 to 6%, preferably from 0.1 to 3%. However, the concentration data for the other constituents of the liquid-crystal mixtures, i.e. of the liquid-crystalline or mesogenic compounds, are indicated without taking into account the concentration of these additives.

The present invention furthermore relates to electro-optical display elements, in particular liquid-crystal, switching and display devices, containing a liquid-crystalline medium, outer plates, electrodes, at least one alignment layer and optionally additional auxiliary layers, where the liquid-crystalline medium comprises at least one compound of the formula I.

The present invention furthermore relates to electro-optical display elements with active-matrix addressing, having nematic or cholesteric phases which comprise at least one compound of the formula I.

Especially preferred are TN, STN, VA, blue mode and IPS displays, in particular those of the active-matrix type. Further preferred are transflective displays.

In the present application and in particular in the examples below, the structures of the liquid-crystal compounds are indicated by means of acronyms, the transformation into chemical formulae taking place in accordance with Tables A and B below. All radicals $C_nH_{2n+1}$ and $C_mH_{2m+1}$ are straight-chain alkyl radicals having n and m carbon atoms respectively. The coding in Table B is self-evident. In Table A, only the acronym for the parent structure is indicated. In individual cases, the acronym for the parent structure is followed, separated by a dash, by a code for the substituents $R^1$, $R^2$, $L^1$ and $L^2$:

| Code for $R^1$, $R^2$, $L^1$, $L^2$ | $R^1$ | $R^2$ | $L^1$ | $L^2$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| n | $C_nH_{2n+1}$ | CN | H | H |
| nN.F | $C_nH_{2n+1}$ | CN | F | H |
| nN.F.F | $C_nH_{2n+1}$ | CN | F | F |

(continued)

| Code for $R^1$, $R^2$, $L^1$, $L^2$ | $R^1$ | $R^2$ | $L^1$ | $L^2$ |
|---|---|---|---|---|
| nF | $C_nH_{2n+1}$ | F | H | H |
| nF.F | $C_nH_{2n+1}$ | F | F | H |
| nF.F.F | $C_nH_{2n+1}$ | F | F | F |
| nOF | $OC_nH_{2n+1}$ | F | H | H |
| nCl | $C_nH_{2n+1}$ | Cl | H | H |
| nCl.F | $C_nH_{2n+1}$ | Cl | F | H |
| nCl.F.F | $C_nH_{2n+1}$ | Cl | F | F |
| $nCF_3$ | $C_nH_{2n+1}$ | $CF_3$ | H | H |
| $nCF_3$.F | $C_nH_{2n+1}$ | $CF_3$ | F | H |
| $nCF_3$.F.F | $C_nH_{2n+1}$ | $CF_3$ | F | F |
| $nOCF_3$ | $C_nH_{2n+1}$ | $OCF_3$ | H | H |
| $nOCF_3$.F | $C_nH_{2n+1}$ | $OCF_3$ | F | H |
| $nOCF_3$.F.F | $C_nH_{2n+1}$ | $OCF_3$ | F | F |
| $nOCF_2$ | $C_nH_{2n+1}$ | $OCHF_2$ | H | H |
| $nOCF_2$.F | $C_nH_{2n+1}$ | $OCHF_2$ | F | H |
| $nOCF_2$.F.F | $C_nH_{2n+1}$ | $OCHF_2$ | F | F |
| nS | $C_nH_{2n+1}$ | NCS | H | H |
| nS.F | $C_nH_{2n+1}$ | NCS | F | H |
| nS.F.F | $C_nH_{2n+1}$ | NCS | F | F |
| rVsN | $C_rH_{2r+1}$-CH=CH-$C_sH_{2s}$ | CN | H | H |
| rEsN | $C_rH_{2r+1}$-O-$C_sH_{2s}$ | CN | H | H |
| nAm | $C_nH_{2n+1}$ | $COOC_mH_{2m+1}$ | H | H |

**Table A:**

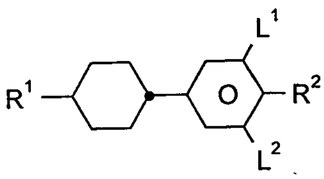

**PCH**

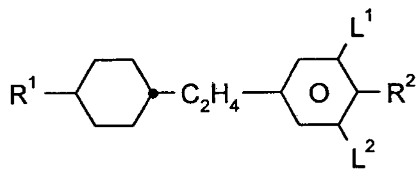

**EPCH**

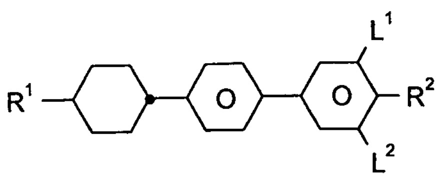

**BCH**

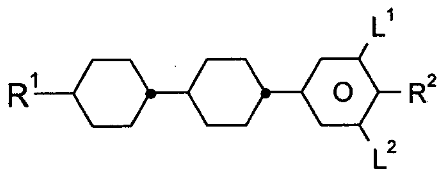

**CCP**

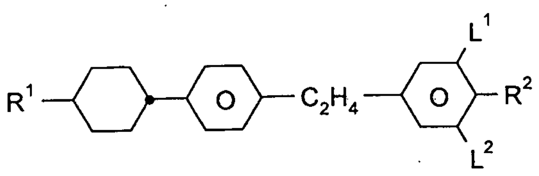

**EBCH**

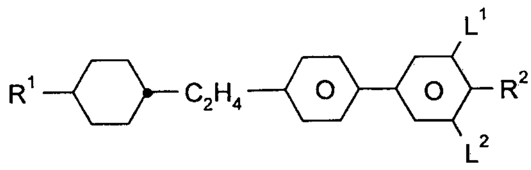

**BECH**

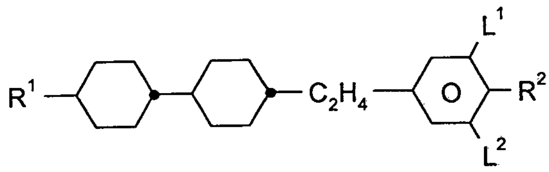

**ECCP**

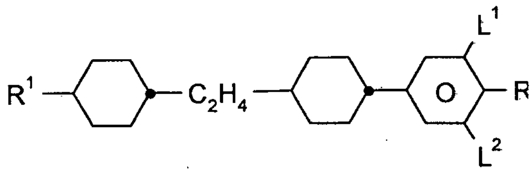

**CECP**

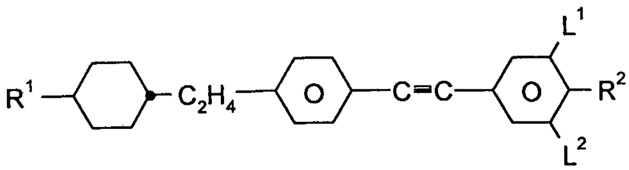

**CEPTP**

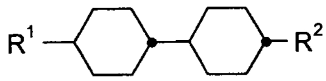

**CCH**

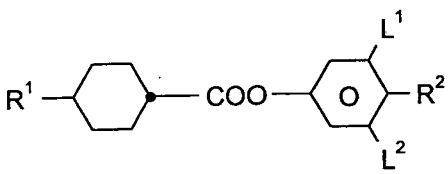

**D**

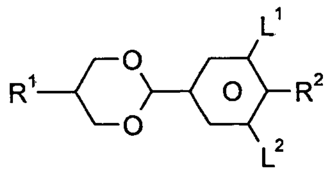

**PDX**

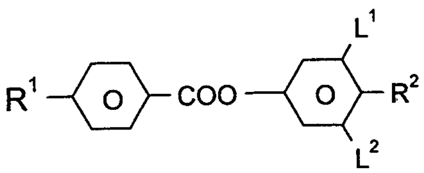

**ME**

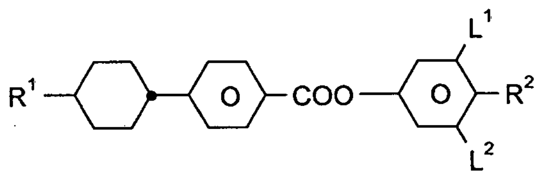

**HP**

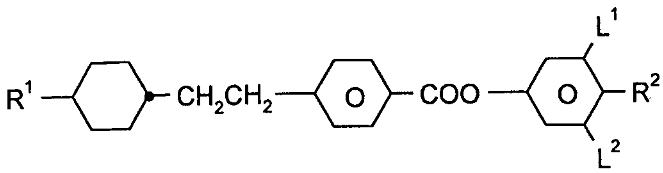

**EHP**

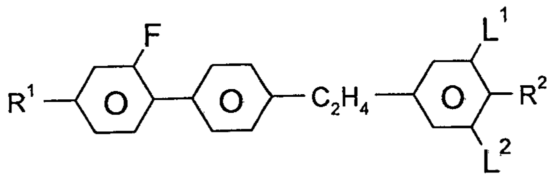

**FET**

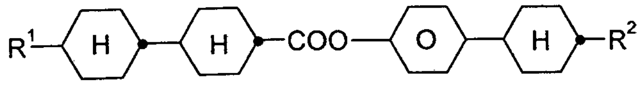

**CCPC**

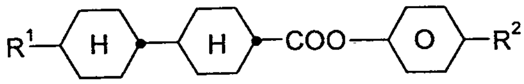

**CP**

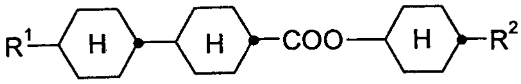

**CH**

**Table B:**

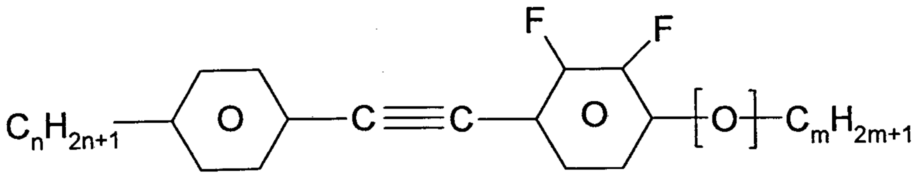

**PTP-n[O]mFF**

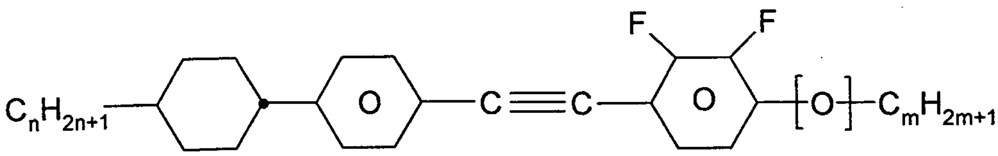

**CPTP-n[O]mFF**

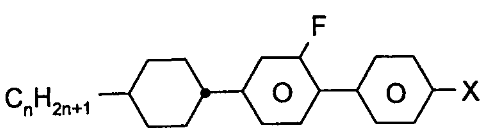

**CGP-n-X**
(X = F, Cl, OCF3)

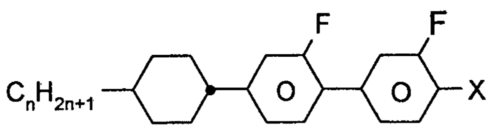

**CGG-n-X**
(X = F, Cl, OCF3)

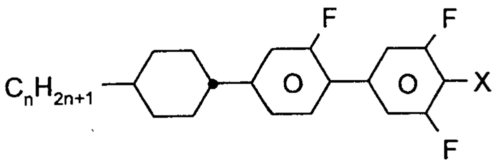

**CGU-n-X**
(X = F, Cl, OCF3)

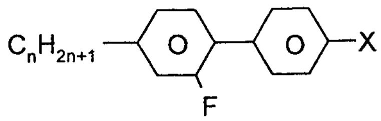

**GP-n-X**
(X = F, Cl, OCF3)

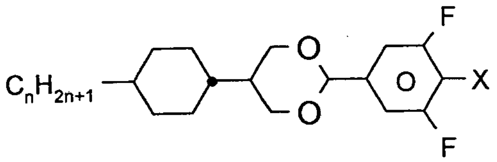

**CDU-n-X**
(X = F, Cl, OCF3)

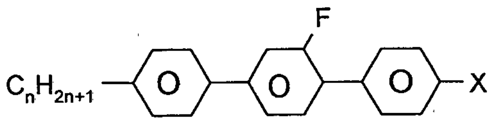

**PGP-n-X**
(X = F, Cl, OCF3)

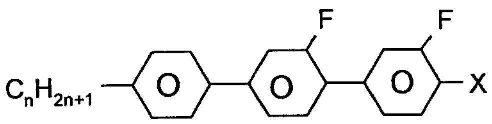

**PGG-n-X**
(X = F, Cl, OCF3)

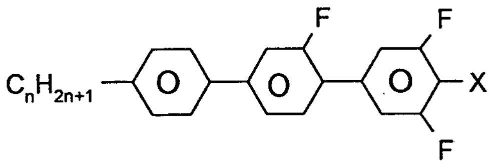

**PGU-n-X**
(X = F, Cl, OCF3)

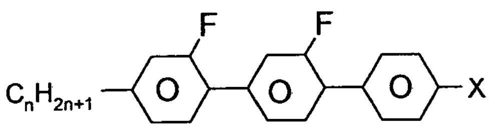

**GGP-n-X**
(X = F, Cl, OCF3)

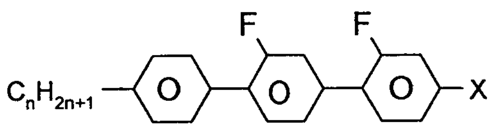

**PGIGI-n-X**
(X = F, Cl, OCF3)

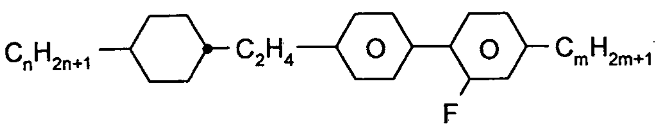

**I-nm**

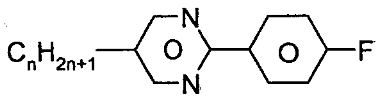

**PYP-n-F**

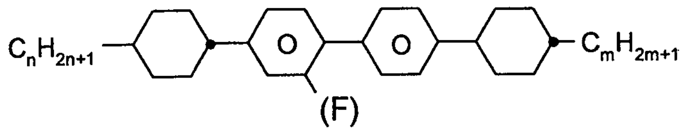

**CBC-nm(F)**

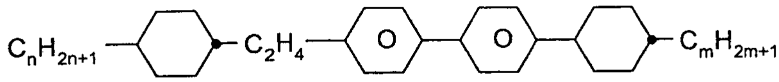

**ECBC-nm**

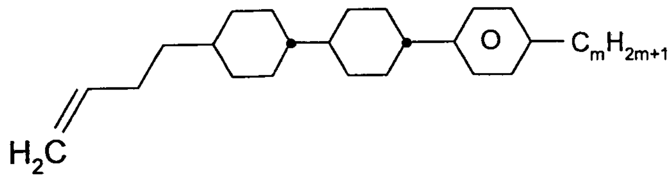

**CCP-V2-m**

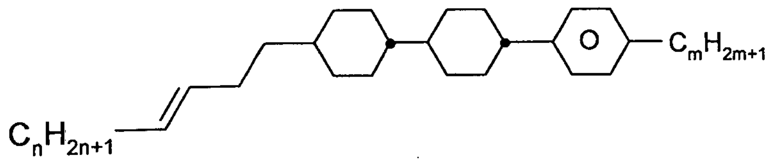

**CCP-nV2-m**

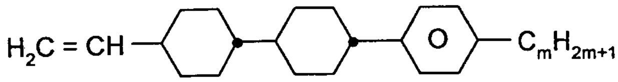

**CCP-V-m**

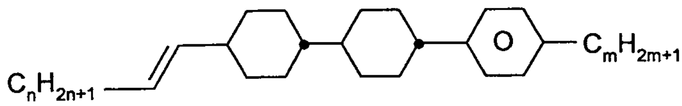

**CCP-nV-m**

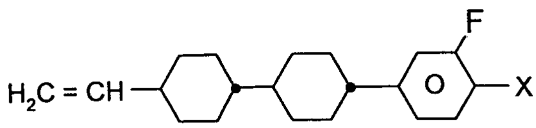

**CCG-V-X**

(X = F, Cl, OCF3)

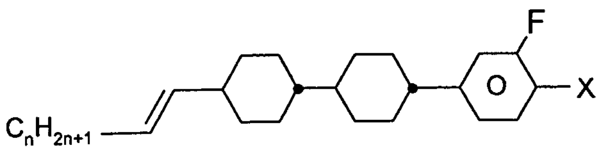

**CCG-nV-X** (X = F, Cl, OCF3)

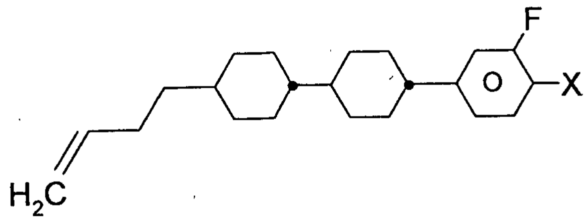

**CCG-V2-X**

(X = F, Cl, OCF3)

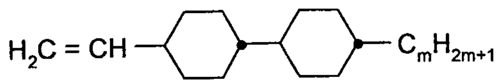

**CC-V-m**

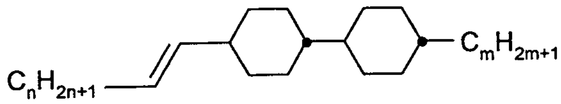

**CC-nV-m**

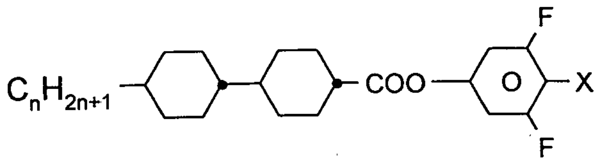

**CCZU-n-X**

(X = F, Cl, OCF3)

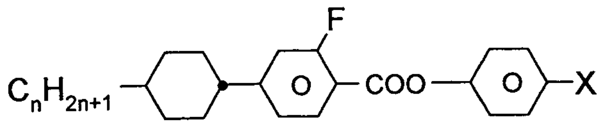

**CGZP-n-X**

(X = F, Cl, OCF3)

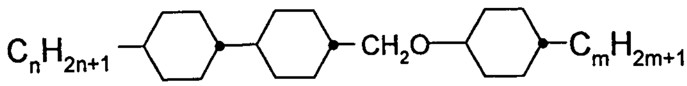

**CCOC-n-m**

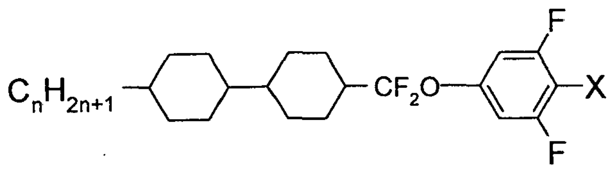

**CCQU-n-X**

(X = F, Cl, OCF3)

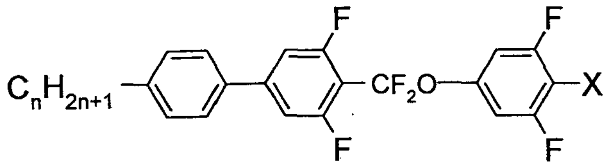

**PUQU-n-X**

(X = F, Cl, OCF3)

The invention is explained in greater detail below with reference to working examples, but without being restricted thereby in any way.

Abbreviations:

DCC *N,N'*-dicyclohexylcarbodiimide
DMAP *N,N*-dimethylaminopyridine.

In the foregoing and the following, all temperatures are given in degrees Celsius, and all percentages are by weight, unless stated otherwise. The following abbreviations are used to illustrate the LC phase behaviour: C, K = crystalline; N = nematic; S = smectic; N*, Ch = chiral nematic or cholesteric; I = isotropic. The numbers between these symbols indicate the phase transition temperatures in degree Celsius. Furthermore, mp is the melting point and cp is the clearing point (in deg.C).

The values of the helical twisting power HTP of a chiral compound in a liquid crystalline host are given according to the equation HTP = $(p*c)^{-1}$ in $\mu m^{-1}$ wherein p is the pitch of the molecular helix, given in μm, and c is the concentration by weight of the chiral compound in the host given in relative values (thus, e.g. a concentration of 1 % by weight is corresponding to a value of c of 0.01). Unless stated otherwise, the HTP values were determined in the the given host mixture at a concentration of 1 % and a temperature of 20 °C. The following abbreviations are used to illustrate the liquid crystalline phase behaviour of the compounds: C = crystalline; N = nematic; S = smectic; N*, Ch = chiral nematic or cholesteric; I = isotropic. The numbers between these symbols indicate the phase transition temperatures in degree Celsius.

## Examples

Synthesis Examples

### Example 1

### $C_1$-Symmetrical Chiral Dopants

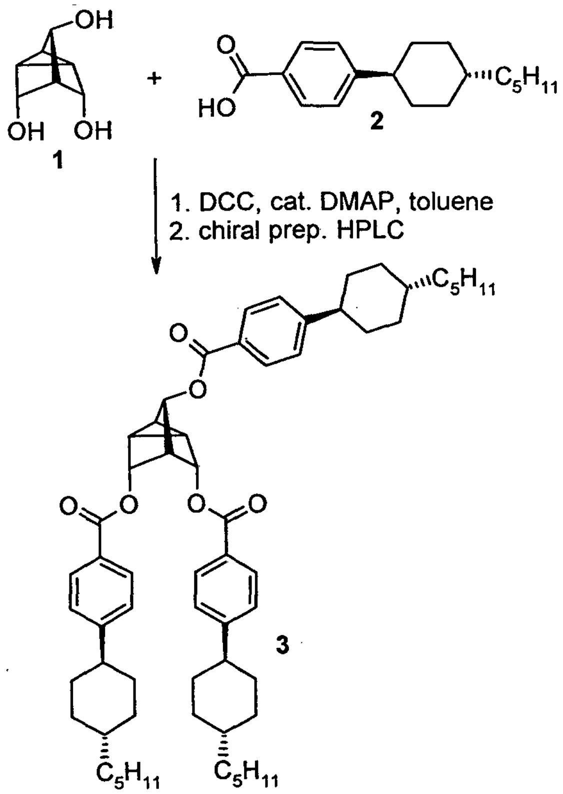


1 + 2

3

**3:** A solution of racemic **rac-1** (400 mg, 2.81 mmol), **2** (2.32 g, 8.44 mmol), DCC (1.95 g, 9.45 mmol) and DMAP (14 mg, 0.12 mmol) in toluene (35 mL) was stirred for 72 h at r.t. The precipitate was filtered off, and washed with a small amount of toluene. The filtrate was evaporated to dryness, chromatographed (silica gel; toluene) and crystallized from ethanol at 5°C to yield racemic ***rac*-3** (1.4 g, 55%; colorless, crystalline solid).

The ***rac*-3** (1.4 g, 1.54 mmol) was submitted to enantiomer separation by chiral HPLC (ChiralCel OJ-H; super-critical $CO_2$/*i*-propanol 9:1) to yield the two enantiomers, which were crystallized from ethanol at 5°C: **3a** (412 mg, 29%) and **3b** (448 mg, 32%) as colorless crystals.

**Example 2**

***C*$_3$-Symmetrical Chiral Dopants**

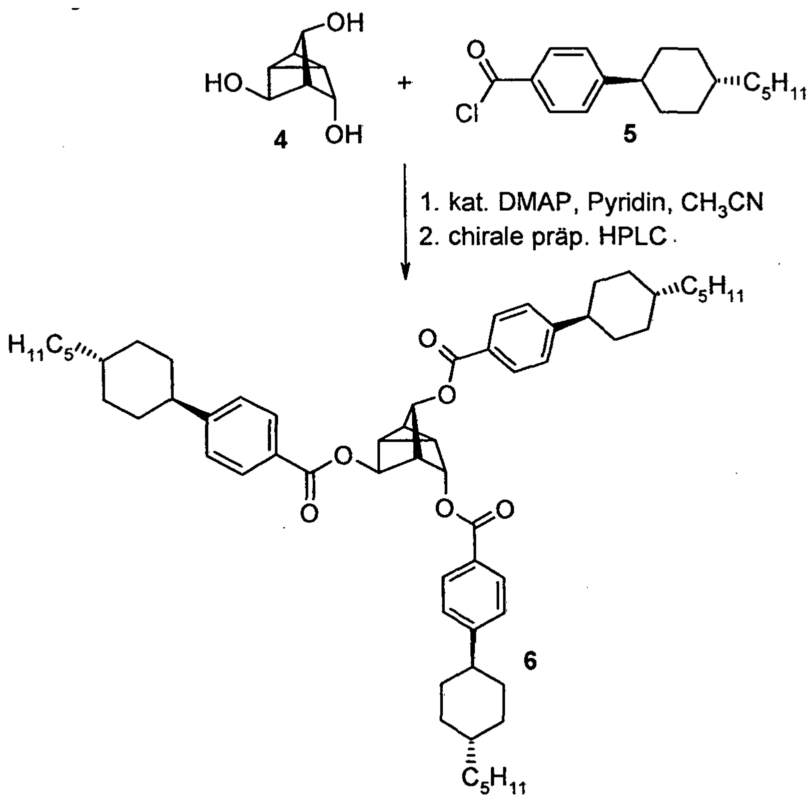


**6**: A solution of ***(S,S,S)*-4** (280 mg, 1.97 mmol), **5** (2.70 g, 9.25 mmol), DMAP (2 mg, 0.02 mmol) and pyridine (0.9 mL) in acetonitrile (40 mL) was stirred for 72 h at r.t. The mixture was diluted with water, extracted three times with $CH_2Cl_2$, and the combined extracts were dried over $Na_2SO_4$. Evaporation of the solvent and subsequent crystallization from ethanol at 5°C furnished crude ***(S,S,S)*-6** (900 mg, 50%) as a colorless powder.

The crude ***(S,S,S)*-6** (676 mg) was submitted to further purification by enantiomer separation using chiral HPLC (ChiralCel OJ-H; supercritical $CO_2$/*i*-propanol 9:1) and subsequent crystallization from ethanol at 5°C to yield pure product ***(S,S,S)*-6** (524 mg, 78%) as colorless crystals. $[\alpha]_D^{25}$ = +155.5° ($CH_2Cl_2$; 12.7 mg/mL).
HTTP: -25

The enantiomer ***(R,R,R)*-6** was prepared in an analogous manner from ***(R,R,R)*-4.**
HTTP: 25

**Preparation of precursors**

**Procedure to obtain racemic $C_3$- and $C_1$-symmetric tricyclo[2.2.1.0$^{2,6}$]heptane-3,5,7-triols, (rac-4, rac-1)**

To the solution of 7-*tert*-butoxy-norbornadiene (20 g, 0.122 mol) in 100 mL acetic acid by cooling with water bath (reaction is exothermic), 23.75 g (0.122 mol) 39%wt solution of peracetic acid in acetic acid was added drop wise during 30 minutes. Then, reaction mixture was stirred 2 hours by r.t. After reaction completed the acetic acid was evaporated, the residue was solved in formic acid (500 mL) and refluxed for 5 days. The obtained black solution was concentrated in vacuo. The black oily residue was solved in methanol (2 × 500 mL) and filtered. To the methanolic solution g (27.64 g, 0.2 mol) potassium carbonate was added. The suspension was allowed to stir for 4 hours. The resulting solution was concentrated. To the solid residue 10% hydrochloric acid solution was added dropwise till acidic. Then water and acetic acid from resulted solution were evaporated in rotary evaporator (temperature in bath 70 °C). The solid residue was washed with methanol (2 × 100 mL) and filtrated from potassium chloride. The methanolic solution was concentrated in rotary evaporator, washed with THF (3 × 100 mL) and decanted. The soluble in THF $C_1$-symmetric triol was exposed to further isomerisation by reflux with formic acid. The insoluble in THF $C_3$-triol was dried in vacuo. Yield of colourless solid *rac*-tricyclo[2.2.1.0$^{2,6}$]heptane-3,5,7-triol after first isomerisation is 32%, after second -10%, after third - 3%. Overall yield for three runs is 45 % (7.79 g). Product can be recrystallized from methanol.

Analysis:

(3*R*,5*R*,7*R*)- and (3*S*,5*S*,7*S*)-tricyclo[2.2.1.0$^{2,6}$]heptane-3,5,7-triol (***rac*-4**)

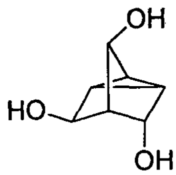

$R_F$ (MeOH : DCM = 1 : 5) = 0.22
M.p.: 208-210 °C (MeOH).
$^1$H NMR (300 MHz, $CDCl_3$): δ 4.49 (s, 3H), 1.66 (m, 1H), 1.50 (s, 3H).
$^{13}$C NMR (75 MHz, $CDCl_3$): δ 75.69 (s), 48.08 (s), 21.54 (s).
ESI-MS found m/z 165.0522 ($M^+$ + Na), calculated: 165.0528
Elem. Anal.: Calc. C 59.14, H 7.09; Found. C 59.03, H 7.22.
Specific rotation (after separation of enantiomers):

(*S*,*S*,*S*)-isomer, $[\alpha]^{25}_D$ = - 68.9° ($CH_3OH$, 10 mg/mL).
(*R*,*R*,*R*)-isomer, $[\alpha]^{25}_D$ = + 68.7° ($CH_3OH$, 10 mg/mL).

(3*S*,5*R*,7*R*)- and (3*R*,5*S*,7*S*)-tricyclo[2.2.1.0$^{2,6}$]heptane-3,5,7-triol (***rac*-1**)

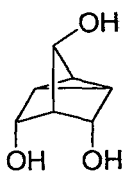

The NMR data agree very well with the literature data of compound obtained by Laihia (K. Laihia, E. Kolehmainen, P. Manttari, R. Ryynanen, M. Messala-Rannanpiha, J. Vepsalainen, J. P. Sundelin. Spectrochim. Acta. Part A 1996, 52, 495-504.).

The triol **rac-1** is used as starting material for Example 1.
$R_F$ (MeOH : DCM = 1 : 5) = 0.42
Mp: 168-171 °C.
$^1$H NMR (300 MHz, $CDCl_3$): δ 4.56 (s, 1H), 3.95 (s, 1H), 3.76 (t, *J* = 1.8 Hz, 1H), 1.84 (s, 1H), 1.67 (dt, *J* = 13.1, 4.8 Hz, 2H), 1.41 (t, *J* = 5.4 Hz, 1H).
$^{13}$C NMR (75 MHz, $CDCl_3$): δ 78.35 (s), 76.60 (s), 72.86 (s), 45.97 (s), 22.21 (s), 21.56 (s), 19.02 (s).
ESI-MS found m/z 165.0522 ($M^+$ + Na), calculated: 165.0528.

**(3*S*,5*S*,7*S*)- and (3*R*,5*R*,7*R*)-tricyclo[2.2.1.02,6]heptane-3,5,7-triyl-tris((S)-2-methoxy-2-phenylacetates), and separation** of **diastereomers**

(*S*)-(+)-2-methoxy-2-phenylacetic acid (MPA) was obtained from mandelic acid and dimethyl sulfate in 37% yield according to the method described by Jones et al. (Jones et al., US 2012/0295329 A1).

(*S*)-(+)-2-methoxy-2-phenylacetic acid (MPA) (12.7 g, 76.5 mmol) was added to a stirred solution of oxalyl chloride (30.0 g, 236.4 mmol) in THF (100 mL) in the presence of a catalytic amount of DMF at ambient temperature. After stirring for 2 hours the reaction mixture was concentrated in vacuo. The thus obtained acid chloride of MPA was purified by distillation (60 °C/ 7·10$^{-2}$ mbar), and then was dissolved in $CH_3CN$ (100 mL) and added dropwise to a suspension of nortricycloheptane-3,5,7-triol (2.5 g, 17.6 mmol) **4**, pyridine (6.04 g, 76.5 mmol) and a catalytic amount of DMAP in $CH_3CN$. The reaction mixture was stirred for additional 3 hours at ambient temperature, while becoming a clear, reddish solution. Completion of the reaction was checked by TLC. Then the solvent was evaporated in vacuo and

the residue was extracted with diethyl ether (3 × 200 mL). The ethereal solution was concentrated and the obtained oily residue was purified by column chromatography. The tris-mandelates (24% of *S*,*S*,*S*-isomer and 30% of *R*,*R*,*R*-isomer) were isolated.

The mixture of tris-mandelates was separated by careful column chromatography.

Analysis:

(3*S*,5*S*,7*S*)-Tricyclo[2.2.1.0$^{2.6}$]heptane-3,5,7-triyl-tris((*S*)-2-methoxy-2-phenylacetate)

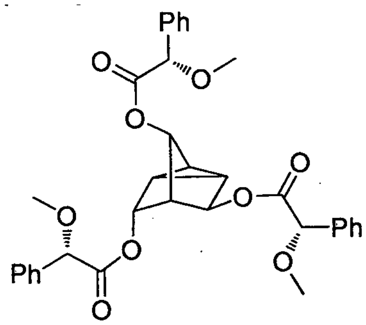

[α]$_D^{20}$ = -37.0° ($CH_3OH$, 10 mg/mL).
M.p. 96-98 °C.
$R_F$ = 0.4 (cyclohexane : ethyl acetate = 2 : 1).
$^1$H NMR (300 MHz, $CDCl_3$): δ 7.47 - 7.28 (m, 15H), 5.10 (s, 3H), 4.74 (s, 3H), 3.40 (s, 9H), 2.34 (s, 1H), 1.53 (s, 3H).
$^{13}$C NMR (75 MHz, $CDCl_3$): δ 170.32 (s), 135.85 (s), 128.84 (s), 128.72 (s), 127.06 (s), 82.34 (s), 77.53 (s), 57.35 (s), 40.73 (s), 17.89 (s)
ESI MS: found 609.2095, calculated ($C_{34}H_{34}NaO_9$) 609.2101.

(3*R*,5*R*,7*R*)-tricyclo[2.2.1.0$^{2,6}$]heptane-3,5,7-triyl-tris((*S*)-2-methoxy-2-phenylacetate)

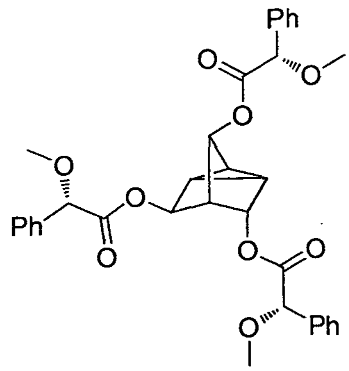

[α]$_D^{20}$ = +116.9° ($CH_3OH$, 10 mg/mL).
M.p. 100-103 °C.
$R_F$ = 0.32 (cyclohexane : ethyl acetate = 2 : 1)
$^1$H NMR (300 MHz, $CDCl_3$): δ 7.46 - 7.22 (m, 15H), 4.94 (s, 3H), 4.71 (s, 3H), 3.38 (s, 9H), 1.88 (s, 1H), 1.85 (s, 3H).
$^{13}$C NMR (75 MHz, $CDCl_3$): δ 170.27 (s), 135.73 (s), 128.86 (s), 128.71 (s), 127.12 (s), 82.24 (s), 77.46 (s), 57.27 (s), 40.27 (s), 18.52 (s).
ESI MS: found 609.2097, calculated ($C_{34}H_{34}NaO_9$) 609.2101.

**Saponification of (3*R*,5*R*,7*R*)- or (3*S*,5*S*,7*S*)-tricyclo[2.2.1.0$^{2,6}$]heptane-3,5,7-triyl-tris((S)-2-methoxy-2-phenylacetate), (*R,R,R*)-4/(*S,S,S*)-4**

To the methanolic solution (100 mL) of one diastereomer of the tris-2-O-methylmandelate esters (3.1 g, 5.28 mmol) potassium carbonate (5.2 g, 37.6 mmol) was added. The suspension was allowed to stir for 4 hours. The resulting solution was concentrated. To the solid residue 10% hydrochloric acid solution was added dropwise till acidic. Then water was evaporated using a rotary evaporator (temperature in bath 70 °C). The solid residue was washed with methanol (2 x 100 mL) and filtrated from potassium chloride. The methanolic solution was concentrated in rotary evaporator, and washed with DCM (3 x 100 mL) and filtrated. The soluble in DCM 2-methoxy-2-phenylacetic acid can be used again for esterification of racemic triol. The insoluble in DCM enantiopure (*R*,*R*,*R*)- or (*S*,*S*,*S*)-triol **4** does not need any additional purification. The yield is 0.72 g (96 %).

The triols **(*R*,*R*,*R*)-4/(*S*,*S*,*S*)-4** are used as starting material for Example 2.

Mixture Examples

To a liquid-crystalline base mixture of the following composition:

| Acronym | Weight-% |
|---|---|
| CCH-301 | 10 |
| CCH-303 | 9 |
| CCPC-33 | 5 |
| CCPC-34 | 5 |
| CCPC-35 | 5 |
| CH-33 | 4 |
| CH-35 | 4 |
| CH-43 | 4 |
| CH-45 | 4 |
| CP-33F | 8 |
| CP-35F | 8 |
| ME-2N.F | 2 |
| ME-3N.F | 2 |
| ME-5N.F | 4 |
| PCH-301 | 16 |
| PYP-5-F | 10 |

is added 1.0% by weight of product from Example 1 or 2, and the helical twisting power HTP of the compositions are determined at 20°C. The helical twisting power HTP of each compound is indicated in the table.

| Compound | HTP |
|---|---|
| Example 1 | 22/-21 (*S*,*R*,*R*-/*R*,*S*,*S*-enantiomer) |
| Example 2 | 25/-25 (*R*,*R*,*R*-/*S*,*S*,*S*-enantiomer) |

## Claims

**1.** Chiral compounds of the general formula I:

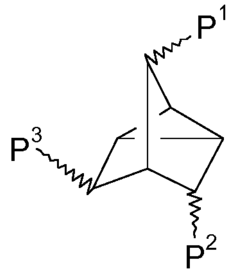

in which:

$P^1$, $P^2$ and $P^3$ independently, are a group R-($A^2$-$Z^2$)$_n$-$A^1$-$Z^1$-,
R is -H, F, Cl, an alkyl or alkenyl radical having from 1 to 12 carbon atoms which is unsubstituted or at least monosubstituted by halogen, and in which one or more non-adjacent -$CH_2$- groups may be replaced by -O- or -S- and/or -C≡C-,
$A^1$, independently, is 1,4-phenylene, in which one, two or three CH groups may be substituted by N and in which, in addition, one or more H atoms may be replaced by F, 1,4-cyclohexylene, in which, in addition, one or two $CH_2$ groups may be replaced by -O-, or 1,4-bicyclo[2.2.2]octanyl,
$A^2$, independently, is 1,4-phenylene, in which one, two or three CH groups may be substituted by N and in which, in addition, one or more H atoms may be replaced by F, 1,4-cyclohexylene, in which, in addition, one or two $CH_2$ groups may be replaced by -O-, or 1,4-bicyclo[2.2.2]octanyl,
$Z^1$, independently is a single bond, -O-, -S-, -CO-, -COO-, -OCO-,-S-CO-, -CO-S-, -O-COO-, -$CH_2$-$CH_2$-, -CH=CH-, -CH=$CR^0$-, -$CR^0$=$CR^{00}$-, -O-$CH_2$-, -$CH_2$-O-, -$CF_2$-O-, -O-$CF_2$-, -$CF_2$-$CF_2$- or -C≡C-, -CH=CH-COO- or -OCO-CH=CH-,
$Z^2$, independently is a single bond, -O-, -S-, -CO-, -COO-, -OCO-,-S-CO-, -CO-S-, -O-COO-, -$CH_2$-$CH_2$-, -CH=CH-, -CH=$CR^0$-, -$CR^0$=$CR^{00}$-, -O-$CH_2$-, -$CH_2$-O-, -$CF_2$-O-, -O-$CF_2$-, -$CF_2$-$CF_2$- or -C≡C-, -CH=CH-COO- or -OCO-CH=CH-,
$R^0$ and $R^{00}$ are independently of each other H or alkyl with 1 to 12 C-atoms, F, Cl or CN, and
n is from 1 to 3.

2. Chiral compounds according to Claim 1, **characterised in that** they are selected of the structures

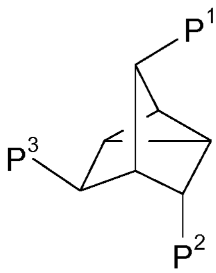 , 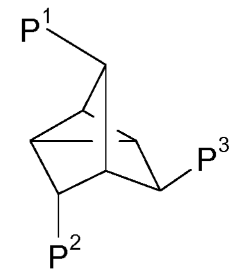 , 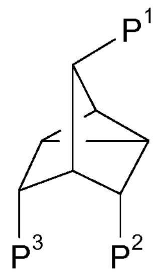 or 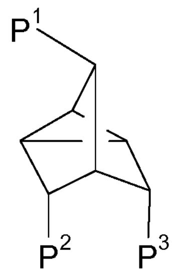

in which $P^1$, $P^2$ and $P^3$ are defined as in claim 1.

3. Chiral compounds according to Claim 1 or 2, **characterised in that** $P^1$, $P^2$ and $P^3$ are identical groups and are not H.

4. Chiral compounds according to one or more of claims 1 to 3, **characterised in that**
n is 1 or 2.

5. Chiral compounds according to one or more of claims 1 to 4, **characterised in that**
$A^1$ and $A^2$ are, independently, selected from the following structures:

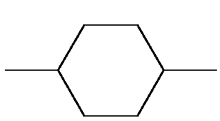 , 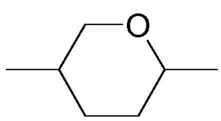 , 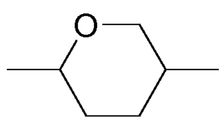 , 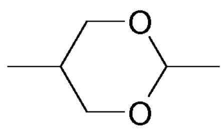 ,

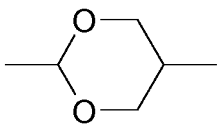 , 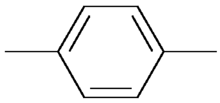 , 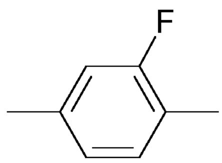 , 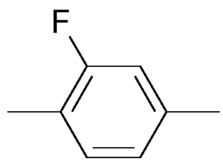 ,

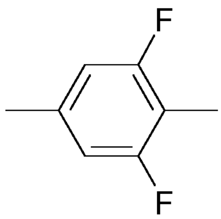 , 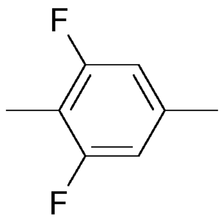

or

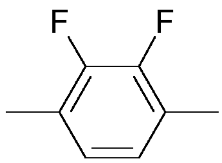 .

**6.** Chiral compounds according to one or more of claims 1 to 5, **characterised in that**

$Z^1$ is independently -O-, -COO- or $-CF_2O-$.

**7.** Chiral compounds according to one or more of claims 1 to 6, **characterised in that**

R is independently an alkyl or alkenyl radical having from 1 to 12 carbon atoms which is unsubstituted or at least monosubstituted by halogen, and in which one or more non-adjacent $-CH_2-$ groups may be replaced by -O-.

**8.** Use of at least one chiral compound according to at least one of the preceding claims in liquid-crystalline mixtures.

**9.** Liquid-crystalline mixture comprising at least one chiral compound according to at least one of Claims 1 to 7.

**10.** Electro-optical display element containing a liquid-crystalline mixture according to Claim 9.

**Patentansprüche**

**1.** Chirale Verbindungen der allgemeinen Formel I:

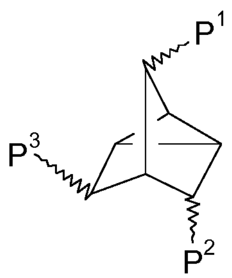

in der:

$P^1$, $P^2$ und $P^3$ unabhängig eine Gruppe $R-(A^2-Z^2)_n-A^1-Z^1-$ sind,
R -H, F, Cl, ein Alkyl- oder Alkenylrest mit 1 bis 12 Kohlenstoffatomen ist, der unsubstituiert oder mindestens

einfach durch Halogen substituiert ist und in dem eine oder mehrere nicht benachbarte Gruppen $-CH_2-$ durch -O- oder -S- und/oder -C≡C-ersetzt sein können,
$A^1$ unabhängig 1,4-Phenylen, in dem eine, zwei oder drei CH-Gruppen durch N substituiert sein können und in dem zusätzlich ein oder mehrere H-Atome durch F ersetzt sein können, 1,4-Cyclohexylen, in dem zusätzlich eine oder zwei $CH_2$-Gruppen durch -O- ersetzt sein können, oder 1,4-Bicyclo[2.2.2]octanyl ist,
$A^2$ unabhängig 1,4-Phenylen, in dem eine, zwei oder drei CH-Gruppen durch N substituiert sein können und in dem zusätzlich ein oder mehrere H-Atome durch F ersetzt sein können, 1,4-Cyclohexylen, in dem zusätzlich eine oder zwei $CH_2$-Gruppen durch -O- ersetzt sein können, oder 1,4-Bicyclo[2.2.2]octanyl ist,
$Z^1$ unabhängig eine Einfachbindung, -O-, -S-, -CO-, -COO-, -OCO-, -S-CO-, -CO-S-, -O-COO-, $-CH_2-CH_2-$, -CH=CH-, $-CH=CR^0-$, $-CR^0=CR^{00}-$, $-O-CH_2-$, $-CH_2-O-$, $-CF_2-O-$, $-O-CF_2-$, $-CF_2-CF_2-$ oder -C≡C-, -CH=CH-COO- oder -OCO-CH=CH- ist,
$Z^2$ unabhängig eine Einfachbindung, -O-, -S-, -CO-, -COO-, -OCO-, -S-CO-, -CO-S-, -O-COO-, $-CH_2-CH_2-$, -CH=CH-, $-CH=CR^0-$, $-CR^0=CR^{00}-$, $-O-CH_2-$, $-CH_2-O-$, $-CF_2-O-$, $-O-CF_2-$, $-CF_2-CF_2-$ oder -C≡C-, -CH=CH-COO- oder -OCO-CH=CH- ist,
$R^0$ und $R^{00}$ unabhängig voneinander H oder Alkyl mit 1 bis 12 C-Atomen, F, Cl oder CN sind, und
n 1 bis 3 ist.

2. Chirale Verbindungen nach Anspruch 1, **dadurch gekennzeichnet, dass** sie aus den Strukturen

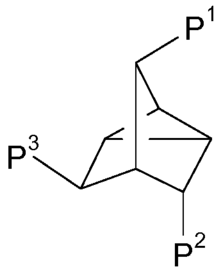 , 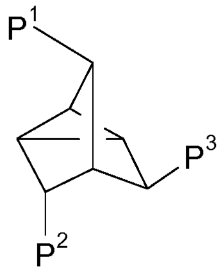 , 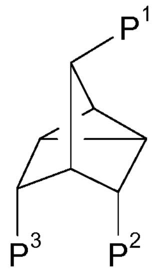 oder 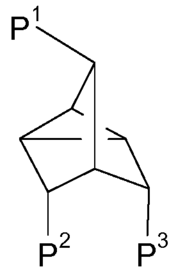

ausgewählt sind, in denen $P^1$, $P^2$ und $P^3$ wie in Anspruch 1 definiert sind.

3. Chirale Verbindungen nach Anspruch 1 oder 2, **dadurch gekennzeichnet, dass** $P^1$, $P^2$ und $P^3$ gleiche Gruppen sind und nicht H sind.

4. Chirale Verbindungen nach einem oder mehreren der Ansprüche 1 bis 3, **dadurch gekennzeichnet, dass** n 1 oder 2 ist.

5. Chirale Verbindungen nach einem oder mehreren der Ansprüche 1 bis 4, **dadurch gekennzeichnet, dass** $A^1$ und $A^2$ unabhängig aus den folgenden Strukturen ausgewählt sind:

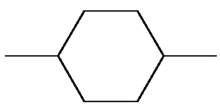 , 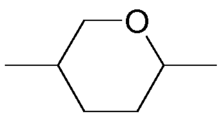 , 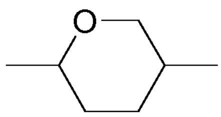 , 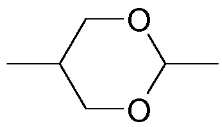 ,

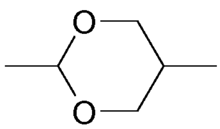 , 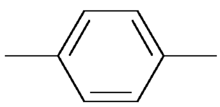 , 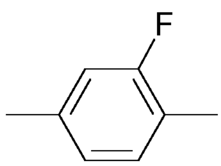 , 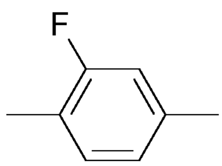 ,

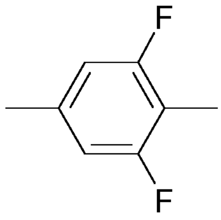
,
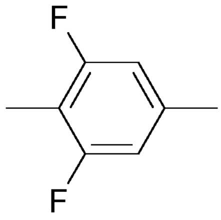

oder

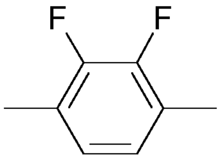
.

6. Chirale Verbindungen nach einem oder mehreren der Ansprüche 1 bis 5, **dadurch gekennzeichnet, dass**

   $Z^1$ unabhängig -O-, -COO- oder $-CF_2O-$ ist.

7. Chirale Verbindungen nach einem oder mehreren der Ansprüche 1 bis 6, **dadurch gekennzeichnet, dass**

   R unabhängig ein Alkyl- oder Alkenylrest mit 1 bis 12 Kohlenstoffatomen ist, der unsubstituiert oder mindestens einfach durch Halogen substituiert ist und in dem ein oder mehrere nicht benachbarte Gruppen $-CH_2-$ durch -O- ersetzt sein können.

8. Verwendung mindestens einer chiralen Verbindung nach mindestens einem der vorhergehenden Ansprüche in flüssigkristallinen Mischungen.

9. Flüssigkristalline Mischung enthaltend mindestens eine chirale Verbindung nach mindestens einem der Ansprüche 1 bis 7.

10. Elektrooptisches Anzeigeelement enthaltend eine flüssigkristalline Mischung nach Anspruch 9.

## Revendications

1. Composés chiraux de la formule générale I :

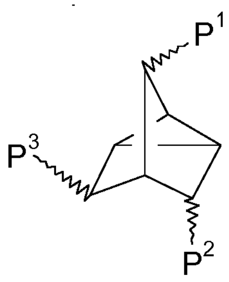

dans laquelle :

   $P^1$, $P^2$ et $P^3$ sont, de manière indépendante, un groupe $R-(A^2-Z^2)_n-A^1-Z^1-$ ;
   R est -H, F, Cl, un radical alkyle ou alkényle qui comporte de 1 à 12 atome(s) de carbone, lequel est non substitué ou au moins monosubstitué par halogène, et où un ou plusieurs groupe(s) -CH2- non adjacents peut/peuvent être remplacé(s) par -O- ou-S- et/ou -C≡C- ;
   $A^1$ est, de manière indépendante, 1,4-phénylène, où un, deux ou trois groupe(s) CH peut/peuvent être substitué(s) par N et où, en outre, un ou plusieurs atome(s) de H peut/peuvent être remplacé(s) par F, 1,4-cyclohexylène, où, en outre, un ou deux groupe(s) $CH_2$ peut/peuvent être remplacé(s) par -O-, ou 1,4-bicyclo[2.2.2]octanyle ;

$A^2$ est, de manière indépendante, 1,4-phénylène, où un, deux ou trois groupe(s) CH peut/peuvent être substitué(s) par N et où, en outre, un ou plusieurs atome(s) de H peut/peuvent être remplacé(s) par F, 1,4-cyclohexylène, où, en outre, un ou deux groupe(s) $CH_2$ peut/peuvent être remplacé(s) par -O-, ou 1,4-bicyclo[2.2.2]octanyle ;

$Z^1$ est, de manière indépendante, une liaison simple, -O-, -S-, -CO-, -COO-, -OCO-, -S-CO-, -CO-S-, -O-COO-, $-CH_2-CH_2-$, -CH=CH-, $-CH=CR^0-$, $-CR^0=CR^{00}-$, $-O-CH_2-$ , $-CH_2-O-$, $-CF_2-O-$, $-O-CF_2-$, $-CF_2-CF_2-$ ou -C≡C-, -CH=CH-COO- ou -OCO-CH=CH- ;

$Z^2$ est, de manière indépendante, une liaison simple, -O-, -S-, -CO-, -COO-, -OCO-, -S-CO-, -CO-S-, -O-COO-, $-CH_2-CH_2-$, -CH=CH-, $-CH=CR^0-$, $-CR^0=CR^{00}-$, $-O-CH_2-$, $-CH_2-O-$, $-CF_2-O-$, $-O-CF_2-$, $-CF_2-CF_2-$ ou -C≡C-, -CH=CH-COO- ou -OCO-CH=CH- ;

$R^0$ et $R^{00}$ sont, de manière indépendante l'un de l'autre, H ou alkyle qui comporte de 1 à 12 atome(s) de C, F, Cl ou CN ; et

n est pris parmi 1 à 3.

**2.** Composés chiraux selon la revendication 1, **caractérisés en ce qu'**ils sont sélectionnés parmi les structures :

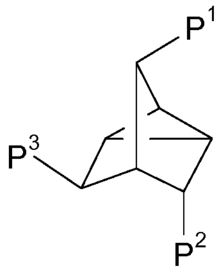 , 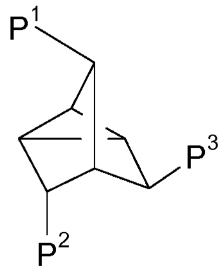 , 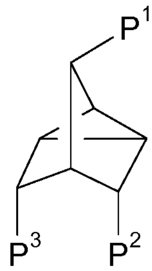 or 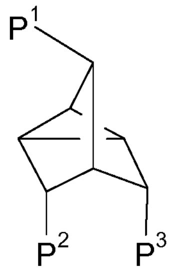

dans lesquelles $P^1$, $P^2$ et $P^3$ sont tels que définis selon la revendication 1.

**3.** Composés chiraux selon la revendication 1 ou 2, **caractérisés en ce que** $P^1$, $P^2$ et $P^3$ sont des groupes identiques et ne sont pas H.

**4.** Composés chiraux selon une ou plusieurs des revendications 1 à 3, **caractérisés en ce que** :
n est 1 ou 2.

**5.** Composés chiraux selon une ou plusieurs des revendications 1 à 4, **caractérisés en ce que** :
$A^1$ et $A^2$ sont, de manière indépendante, sélectionnés parmi les structures qui suivent :

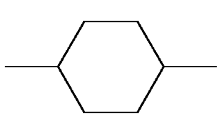 , 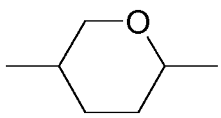 , 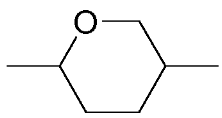 , 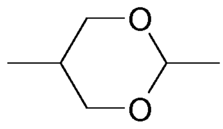 ,

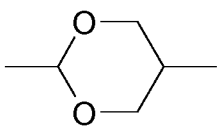 , 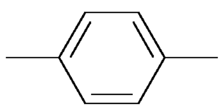 , 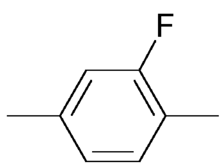 , 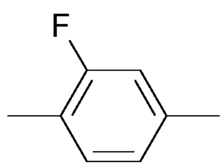 ,

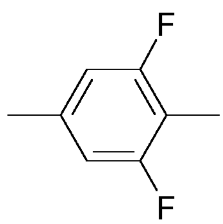 , 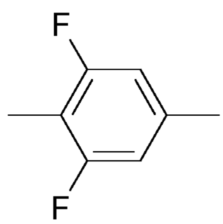

ou

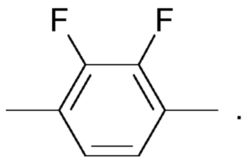

6. Composés chiraux selon une ou plusieurs des revendications 1 à 5, **caractérisés en ce que** :

   $Z^1$ est, de manière indépendante, -O-, -COO- ou $-CF_2O-$.

7. Composés chiraux selon une ou plusieurs des revendications 1 à 6, **caractérisés en ce que** :

   R est, de manière indépendante, un radical alkyle ou alkényle qui comporte de 1 à 12 atome(s) de carbone, lequel est non substitué ou au moins monosubstitué par halogène, et où un ou plusieurs groupe(s) $-CH_2-$ non adjacents peut/peuvent être remplacé(s) par -O-.

8. Utilisation d'au moins un composé chiral selon au moins l'une des revendications qui précèdent dans des mélanges de cristaux liquides.

9. Mélange de cristaux liquides comprenant au moins un composé chiral selon au moins l'une des revendications 1 à 7.

10. Élément d'affichage électro-optique contenant un mélange de cristaux liquides selon la revendication 9.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 4322905 A1
- EP 0609566 A1
- US 20120295329 A1, Jones


### Non-patent literature cited in the description


- **A. E. HOURS ; J. K. SNYDER.** PtCl2-Promoted cyclopropane opening in [4+2+2] homo Diels-Alder cycloadducts. *TETRAHEDRON LETTERS,* 2006, vol. 47, 675-678
- Houben-Weyl, Methoden der Organischen Chemie [Methods of Organic Chemistry. Georg-Thieme-Verlag
- **Y. YABE et al.** *SID 1991 Digest,* 1991, 261-264
- **K. LAIHIA ; E. KOLEHMAINEN ; P. MANTTARI ; R. RYYNANEN ; M. MESSALA-RANNANPIHA ; J. VEPSALAINEN ; J. P. SUNDELIN.** *Spectrochim. Acta. Part A,* 1996, vol. 52, 495-504